# EUROPÄISCHE PATENTSCHRIFT

(11) **EP 0 615 441 B1**
(45) Veröffentlichungstag und Bekanntmachung des Hinweises auf die Patenterteilung: **26.06.1996**
(21) Anmeldenummer: 92924553.8
(22) Anmeldetag: 04.12.1992
(51) Int. Cl.: A61K 9/10, A61K 9/51, A61K 31/19

(54) **EIN IBUPROFEN ENTHALTENDES AKUT-ARZNEIMITTEL UND SEINE VERWENDUNG**
IMMEDIATE-EFFECT IBUPROFEN-CONTAINING MEDICAMENT AND ITS USE
MEDICAMENT A EFFET IMMEDIAT CONTENANT DE L'IBUPROFENE ET SON UTILISATION

(30) Priorität: 05.12.1991 DE 4140179
(43) Veröffentlichungstag der Anmeldung: 21.09.1994
(73) Patentinhaber: ALFATEC-PHARMA GmbH, D-69120 Heidelberg (DE); PAZ ARZNEIMITTEL- ENTWICKLUNGSGESELLSCHAFT MBH, D-65933 Frankfurt (DE)
(72) Erfinder: WUNDERLICH, Jens-Christian, D-6900 Heidelberg (DE); SCHUSTER, Otto, D-6232 Bad Soden (DE); LUKAS, Helmut, D-6078 Neu-Isenburg (DE); SCHICK, Ursula, D-6908 Wiesloch (DE)
(74) Vertreter: Fürniss, Peter, Dr.
(86) Internationale Anmeldenummer: DE9201016
(87) Internationale Veröffentlichungsnummer: WO9310762

(56) Entgegenhaltungen:
- EP-A- 0 267 321
- EP-A- 0 275 796
- EP-A- 0 282 020
- EP-A- 0 290 168
- EP-A- 0 349 428
- WO-A-90/14081
- WO-A-91/06292
- FR-A- 2 608 427
- CHEMICAL ABSTRACTS, vol. 115, no. 18, 4. November 1991, Columbus, Ohio, US; abstract no. 189667e, BODMEIER R. ET AL 'Spontaneous formation of drug- containing acrylic nanoparticles' Seite 420 ;Spalte 2 ;

## Beschreibung

Die Erfindung betrifft ein Akut-Arzneimittel zur Behandlung von schmerzhaften und/oder entzündlichen, sowie fieberhaften Erkrankungen, das Ibuprofen neben üblichen pharmazeutischen Trägern und Hilfsstoffen enthält, das dadurch gekennzeichnet ist, daß das Ibuprofen in Form eines pharmazeutisch applizierbaren Nanosols vorliegt, das als Träger im wesentlichen Gelatine, ein Kollagenhydrolysat oder ein Gelatinederivat enthält.

Die Erfindung betrifft weiterhin ein solches Arzneimittel, das Ibuprofen als Akutform enthält. Schließlich betrifft die Erfindung die Verwendung eines pharmazeutisch applizierbaren Nanosols von Ibuprofen zur Herstellung von Arzneimitteln mit akuter analgetischer und/oder antirheumatischen Wirkung.

Zu den unterschiedlichen Krankheitsbildern des rheumatischen Formenkreises zählt man unter anderem Erkrankungen wie rheumatoide Arthritis, Osteoarthritis und Polymyalgia rheumatica. Die symptomatische Therapie mit nichtsteroidalen Antirheumatica bewirkt dabei in erster Linie die Beseitigung von Schmerzen und weiterhin die Hemmung entzündlicher Prozesse der betroffenen Gelenke. Die dritte Wirkkomponente betrifft die Antiphlogese, womit eine Verbesserung der Beweglichkeit, sowie das Aufhalten des progredienten Krankheitsverlaufs einhergeht.

Zur Gruppe der nichtsteroidalen Antirheumatika gehören eine Vielzahl verschiedener Sustanzklassen, denen überwiegend der Säurecharakter der einzelnen Wirkstoffe gemeinsam ist.

Ibuprofen (2-(4-Isobutylphenyl)propionsäure C₁₃H₁₈O₂) der folgenden Struktur 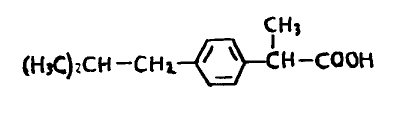
ist ein bekanntes Schmerz- und Rheumamittel aus der Gruppe der 2-Anylpropionsäuren. Normalerweise fällt Ibuprofen bei der chemischen Synthese als Racemat an. Es ist bekannt, daß das Racemat beträchtliche unerwünschte Nebenwirkungen aufweist. Die pharmakologische Forschung der letzten Jahre belegt, daß der Einsatz der enantiomerreinen Ibuprofene große Vorteile hinsichtlich der Reduzierung unerwünschter Wirkungen sowie eine Senkung der Dosis mit sich bringt. Die neuentwickelte großtechnische Herstellung von reinen Enantiomeren ermöglicht für diese Stoffe nun nicht nur einen umfassenden Einsatz in der Therapie, sondern auch neue Möglichkeiten der Kombination der Enantiomeren untereinander. Als moderne Schmerz- und Rheumamittel gewinnen die enantiomerreinen Wirkstoffe zunehmend an Bedeutung. Führende Pharmakologen stellen die Forderung, von racemischen Stoffgemischen nur das Eutomer, d.h. das Enantiomer mit der erwünschten Wirkung einzusetzen und das Distomer mit unerwünschter Wirkung zu entfernen. Im Falle von Ibuprofen lassen sich beiden Enantiomeren bestimmte Wirkungen zuschreiben: S-Ibuprofen weist in erster Linie periphere Wirkung auf, während das Enantiomere R-Ibuprofen offensichtlich zentrale Wirksamkeit besitzt.

Ein weiterer interessanter Effekt gilt für den Einsatz von Pseudoracematen von Ibuprofen:
Von dem strukturverwandten 2-Arylpropionsäurederivat Flurbiprofen ist bekannt, daß sich eine künstliche Mischung (Pseudoracemat, gemischt aus je 50% des S- und des R-Enantiomeren) bezüglich ihrer Auflösungsgeschwindigkeit (Freisetzung) in wäßrigem Milieu anders verhält, als das aus je 50% S- und R-Enantiomeren bestehende, bei der üblichen Synthese anfallende racemische Flurbiprofen. Tatsächlich ist die Auflösungsgeschwindigkeit des Pseudoracemats wesentlicher höher als die des Racemats.

Röntgendiffraktometrische Untersuchungen haben für Ibuprofen ergeben, daß die reinen Enantiomere jeweils in anderen Kristallgittern als das Racemat auskristallisieren. Weiterhin lassen sich stärkere Unterschiede im Schmelzpunkt (Racemat: 75-77,5°C, S-Ibuprofen: 51-52°C), in der Löslichkeit (Racemat: 4,8 mg in 100 ml Wasser bei 37°C, im Gegensatz dazu unter gleichen Bedingungen S-Ibuprofen 11,8 mg), sowie in der Lösegeschwindigkeit zwischen dem Racemat, den einzelnen Enantiomeren und dem Pseudoracemat feststellen, so daß der oben beschriebene Effekt bei Ibuprofen noch stärker als bei Flurbiprofen ausgeprägt ist. Als Folge davon zeigen Untersuchungen, daß nach Applikation des Pseudoracemats wesentlich höhere Blutspiegel erreicht wurden, als nach Gabe des Racemats.

Trotz aller Bemühungen ist es jedoch noch nicht gelungen, eine galenische Formulierung für Ibuprofen zu entwickeln, die alle Anforderungen an eine schnell anflutende Akutform erfüllt.

Ibuprofen, seine Enantiomere, das Pseudoracemat und Mischungen der Enantiomeren in unterschiedlichen Anteilen gehören mit der oben beschriebenen Löslichkeit zu den schwerlöslichen Stoffen. Es ist nach den allgemeinen Resorptionstheorien bekannt, daß Wirkstoffe im Körper nur in gelöster und undissoziierter Form resorbiert werden. Für die galenische Formulierung zu einer Arzneiform muß daher dieses Faktum berücksichtigt werden, denn aus der Schwerlöslichkeit resultiert eine problematische Bioverfügbarkeit.

Eine gängige Methode, dieses Problem zu überwinden, stellt die Mikronisierung von Wirkstoffen dar. Hierbei versucht man gemäß der Noyes-Whitney-Gleichung, die Partikelgröße zu verringern, was eine Vergrößerung der effektiven Wirkstoffoberfläche A bewirkt. Dadurch wird die Bioverfügbarkeit verbessert. So ist es auch für schwerlösliche Analgetika/Antirheumatika üblich, mikronisierte Pulver einzusetzen.

Die Technik der Mikronisierung ist energie- und kostenaufwendig und es treten Probleme mit den sehr feinen Stäuben auf, wie z.B. die Gefahr einer Staubexplosion und die Gefahr der Staubinhalation durch das Personal, was umfangreiche Schutzmaßnahmen erfordert. Das Pulver selbst ist aufgrund elektrostatischer Aufladung bezüglich der Fließeigenschaften schwierig zu verarbeiten und ist meist schlecht benetzbar (Aerophilie). Infolge der hohen Produktionskosten liegen die Preise für mikronisiertes Pulver wesentlich höher als für herkömmliche Pulver, zumal bei der Verarbeitung der reinen Enantiomere des Ibuprofens aufgrund ihrer niedrigen Schmelzpunkte von 51-52°C unter Kühlung mikronisiert werden muß.

Um jedoch im Dünndarm, wo Ibuprofen bevorzugt resorbiert wird, eine ausreichende Lösungsgeschwindigkeit zu gewährleisten, stellt die Mikronisation bis jetzt eine üblicherweise angewandte Methode dar.

Es hat sich gezeigt, daß die Minimierung der gastrointestinalen Nebenwirkungen nur durch den Einsatz von enantiomerreinen Wirkstoffen allein noch nicht zufriedenstellend ist, denn neben der Schwerlöslichkeit besitzt Ibuprofen, sowohl das Racemat als auch die reinen Enantiomere oder Mischungen davon, als Wirkstoffsäure eine weitere nachteilige Eigenschaft: Gelöste oder solubilisierte Anteile können im sauren Magen-Milieu rekristallisieren. Solche Kristalle können unter Umständen Magenirritationen hervorrufen. Darüber hinaus muß man annehmen, daß ein rekristallisierter Anteil für die Resorption nicht mehr zur Verfügung steht, da bei pH 1 im Magensaft das Lösungsgleichgewicht der Wirkstoffsäure zum größten Teil auf der Seite des ungelösten Stoffes liegt.

Aus diesem Grunde werden Ibuprofen-Tabletten üblicherweise magensaftresistent überzogen. Abgesehen von dem technologischen Aufwand dieser Überzugsverfahren muß die verschlechterte Qualität der gängigen Überzüge nach Lagerung beachtet werden.

Dies bedeutet für Ibuprofen, daß die oben beschriebenen Rekristallisationsprozesse bei unzureichenden magensaftresistenten Überzügen einsetzen und man mit einer erheblichen Einschränkung der Wirksamkeit und den beschriebenen Nebenwirkungen rechnen muß.

Die in den letzten Jahren neu gewonnenen Erkenntnisse über Magenphysiologie und Magenmotorik belegen, daß monolithische Arzneiformen, wie nicht zerfallende magensaftresistent überzogene Tabletten und Dragees, eine längere Verweildauer im Magen in Abhängigkeit von seinem Füllungszustand aufweisen als Partikel mit einer Größe unterhalb 2 mm. Es wird verständlich, daß solch große Formlinge dann im Magen verbleiben, wobei Magenverweilzeiten von bis zu 10 Stunden auftreten können. Der Beginn des therapeutischen Effektes ist aufgrund dieser Tatsache nahezu nicht vorherzubestimmen und eine solche Arzneiform muß galenisch als nicht mehr zeitgemäß betrachtet werden. Da sie bereits "slow release" aufgrund langer Magenverweilzeiten zeigen, darf man definitionsgemäß solche Formulierungen eigentlich nicht mehr als Akutform bezeichnen.

Das Problem der Schwerlöslichkeit von Ibuprofen wird im Stand der Technik weiterhin dadurch gelöst, daß man es in ein gut wasserlösliches Salz überführt. Racemisches Ibuprofen wird beispielsweise als wasserlösliches Lysinsalz angeboten, was im Vergleich zur freien Wirkstoffsäure einen schnelleren und signifikant höheren Blutspiegelmaximalwert cₘₐₓ bewirken soll. Handelsübliche Ibuprofen-Lysinat enthaltende Tabletten sind nicht magensaftresistent überzogen, sodaß das Auskristallisieren des Wirkstoffes im sauren Magen-Milieu mit allen oben genannten Nachteilen nicht verhindert werden kann. Daher ist auch keine schnellere Anflutung des Wirkstoffes in der Biophase unterhalb einer Stunde zu erwarten.

Ibuprofen wird erst im oberen Dünndarm resorbiert, was einen späten Wirkeintritt zur Folge hat. Bei einem Schmerzmittel ist jedoch eine schnelle Wirkung erwünscht. Im Falle von racemischem Ibuprofen besagt beispielsweise der pharmakokinetische Parameter tₘₐₓ von 1-2 Stunden, daß nach dieser Zeit das Blutspiegelmaximum erreicht ist. Es liegen Studien vor, wo bei zwei von 8 untersuchten Probanden therapeutische Konzentrationen erst nach mehr als 4 Stunden erreicht werden. In Kenntnis dieser Fakten wird es leicht verständlich, daß ein unter Schmerzen leidender Patient noch vor dem Wirkungseintritt der ersten Dosis eine zweite oder dritte Dosis einnimmt, weil der gewünschte analgetische Effekt vermeintlich ausbleibt. So setzt sich der Patient der Gefahr einer Überdosierung aus.

Betrachtet man zu diesem Faktor noch die Problematik der langen Magenverweildauer von magensaftresistent überzogenen Tabletten, so kann für tₘₐₓ im Falle des Ibuprofens ein noch größerer Wert resultieren und der Patient wird noch eher zu einer Mehrfacheinnahme verleitet. Diese Problematik tritt verstärkt bei gefülltem Magen auf, wodurch sich die Magenverweilzeit zusätzlich verlängern kann.

Daher kann der relativ schnelle Wirkeintritt einer galenisch gut formulierten Acetylsalicysäuretablette hinsichtlich der pharmakokinetischen Parameter tₘₐₓ und cₘₐₓ bei Ibuprofen mit klassischer Galenik nicht erwartet werden.

J.J. Marty et al., Pharm. Acta Helv. 53, 1 (1978) S. 17-23 beschreibt die Herstellung von Gelatine-Nanopartikeln, in die auch Wirkstoffe eingeschlossen werden können. Bei der Herstellung dieser Gelatine-Nanopartikel wird zur Desolvatation und Resolvatation eine pH-Justierung vorgesehen. Eine Überführung des Arzneimittels in Nanopartikel wird nicht offenbart.

Der vorliegenden Erfindung liegt daher die Aufgabe zugrunde, ein Arzneimittel sowie ein Verfahren zu seiner Herstellung für die schnelle Freisetzung und Anflutung von Ibuprofen zu entwickeln, die die oben zum Stand der Technik genannten Nachteile weitgehend vermeiden.

Diese Aufgabe wird erfindungsgemäß durch ein Akut-Arzneimittel gemäß Patentanspruch 1 gelöst. Diese Aufgabe wird weiterhin durch die Verwendung eines pharmazeutisch applizierbaren Nanosols von Ibuprofen gemäß Patentanspruch 20 bis 22 gelöst.

Bevorzugte Ausführungsformen der Erfindung werden in den Unteransprüchen genannt und beansprucht. Im Rahmen der vorliegenden Erfindung werden darüberhinaus völlig neuartige Kombinationen von Akut- und Retardformen möglich. Übliche Einzeldosierungen für das Ibuprofen-Racemat liegen bei 200mg bis 800mg, im Falle des S-Ibuprofens sind 50 mg bis 400mg üblich. Ibuprofen liegt im Rahmen der vorliegenden Erfindung entweder als Racemat, als racemisches Gemisch mit seinen Enantiomeren, als Pseudoracemat (Mischung von gleichen Anteilen S- und R-Ibuprofen) oder in Mischung unterschiedlicher Anteile von S- und R-Ibuprofen im Bereich zwischen reinem S- und reinem R-Ibuprofen vor.

In der internationalen (PCT-)Patentanmeldung PCT/DE92/01010 vom 04.12.1992 mit dem Titel "Pharmazeutisch applizierbares Nanosol und Verfahren zu seiner Herstellung" der ALFATEC-Pharma GmbH entsprechend dem deutschen Patents P 4140195, angemeldet am 05.12.1991, deren Inhalt auch zum Inhalt der vorliegenden Patentanmeldung gemacht wird, werden Nanosole und Verfahren zu ihrer Herstellung beschrieben, die es ermöglichen, kolloid-disperse Lösungen von in Wasser schwer löslichen Wirkstoffen durch Gelatine, Kollagenhydrolysate oder Gelatinederivate zu stabilisieren, wenn man den isoionischen Punkt (=Ladungsausgleich) zwischen Gelatine und den auf der Oberfläche geladenen Wirkstoffpartikeln wenigstens annähernd einstellt. Dabei bringt man das System Wirkstoffpartikel/Gelatine dadurch zum Ladungsausgleich, daß die Oberflächenladung der Partikel durch entsprechende Gegenladung der Gelatinemoleküle kompensiert wird. Erreicht wird dies durch Einstellung einer bestimmten Ladung auf den Gelatinemolekülen, die in Abhängigkeit zu ihrem isoelektrischen Punkt und dem pH-Wert der Lösung steht.
Fig. 1 zeigt die Auflösungsprofile von einer erfindungsgemäßen Tablette (Bsp. 1) und von einer herkömmlichen Filmtablette,
Fig. 2 zeigt die Plasmakonzentrations-Zeitverläufe von einer erfindungsgemäßen Nanosol-Tablette und von einer wäßrigen Suspension

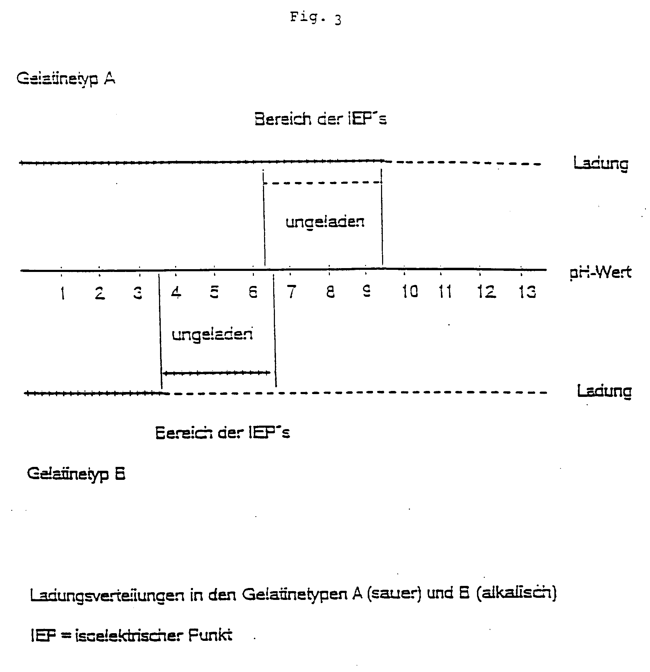

Fig. 3 zeigt eine schematische Darstellung der einstellbaren Ladungszustände von Gelatine in Abhängigkeit vom pH-Wert und IEP, wobei der IEP je nach Herstellungsart zwischen 3,5 und 9,5 liegen kann. Unterhalb von pH 3,5 sind fast alle Gelatinetypen positiv geladen. Im basischen Bereich oberhalb von pH 9,5 sind alle Gelatinetypen negativ geladen.

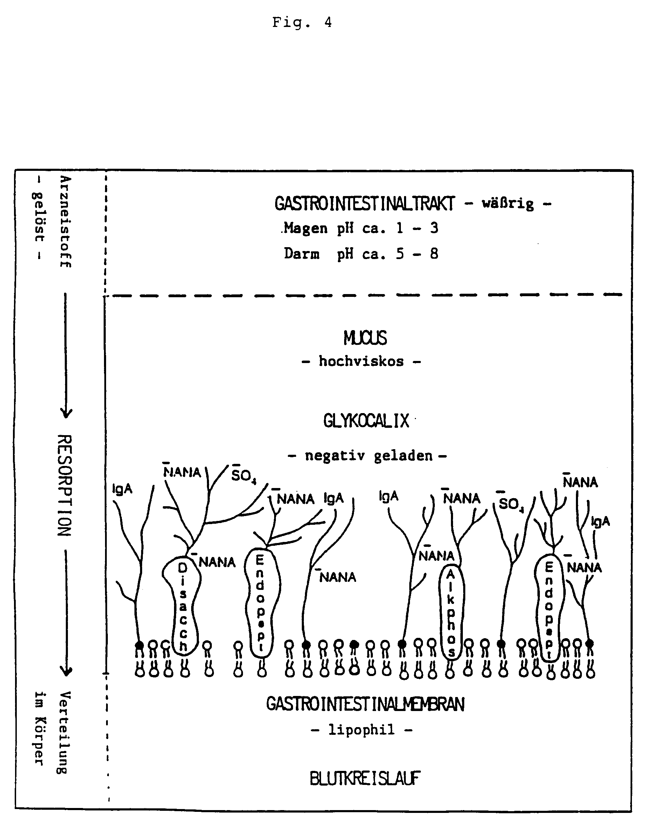

Fig. 4 zeigt den Mechanismus der passiven Arzneistoff-Resorption im Gastrointestinal-Trakt.

Erfindungsgemäß wird daher die Tatsache ausgenutzt, daß Gelatinen, Kollagenhydrolysate oder Gelatinederivate (nahezu unabhängig von der Viskosität) dann zu einem stabilen kolloid-dispersen Systems in Nanosolform führen, wenn der isoionische Ladungszustand zwischen Arzneistoffpartikel und Gelatine, Kollagenhydrolysat oder Gelatinederivat vorliegt.

Dagegen wurde Gelatine nach dem Stand der Technik nur zur Stabilisierung eines anorganischen, kolloid-dispersen Systems eingesetzt. So beschreibt z.B. das DAB 9 eine kolloidale Injektionslösung von radioaktivem Gold, die mit Gelatine hergestellt ist. Man stellte sich dabei lediglich vor, daß sich Makromolekül als "Kittsubstanz" zwischen den einzelnen Kolloidpartikeln befinde und so eine Teilchenaggregation verhindert werde.Dagegen war über den Stabilisierungsmechanismus, z.B. für Arzneistoffe, bisher nichts bekannt.

Die internationalen (PCT-)Patentanmeldungen vom 04.12.1992 der ALFATEC-Pharma GmbH und der PAZ Arzneimittelentwicklungsgesellschaft mbH entsprechend dem genannten deutschen Patent P 4140195, angemeldet am 05.12.1991, betreffen die Akutform von S- und R-Ibuprofen (P 4140179.4-41), die Retardform von S- und R-Ibuprofen (P 4140172.7-41), die Akutform von S- und R-Flurbiprofen (P 4140184.0-41) und die Retardform von S- und R-Flurbiprofen (P 4140183.2-41).

Hand. Durch eine kontrollierte Resorption der Wirkstoffe bereits im Magen kann die aufgrund ihrer Schwerlöslichkeit bisher als problematisch eingestufte Anflutungsgeschwindigkeit und Bioverfügbarkeit von Ibuprofen überraschenderweise erheblich verbessert werden.

Um die physiologischen Hintergründe der Resoprtion von Arzneistoffen im allgemeinen und die verbesserte Resorptionsquote der erfindungsgemäßen Nanosole ausreichend zu erläutern, ist zunächst eine Betrachtung zum Mechanismus der physiologischen Resorption von Arzneistoffen erforderlich, wie er auch in einschlägigen Publikationen dargestellt wird. Allerdings ist die vorliegende Erfindung weder an den folgenden Versuch einer wissenschaftlichen Erklärung der erfindungsgemäß auftretenden Phenomene gebunden noch kann sie hierdurch eingeschränkt werden.

Die passive Arzneistoffresorption erfolgt nach heutigem Erkenntnisstand (Theorie nach Brodie et al.), wenn folgende Bedingungen vorliegen:
a) die Gastrointestinalmembran wirkt als Lipidbarriere,
b) der Arzneistoff wird nur in gelöster und ungeladener, d.h. nichtionisierter Form aufgenommen,
c) saure Arzneistoffe werden bevorzugt im Magen, basische Arzneistoffe bevorzugt im Darm resorbiert.

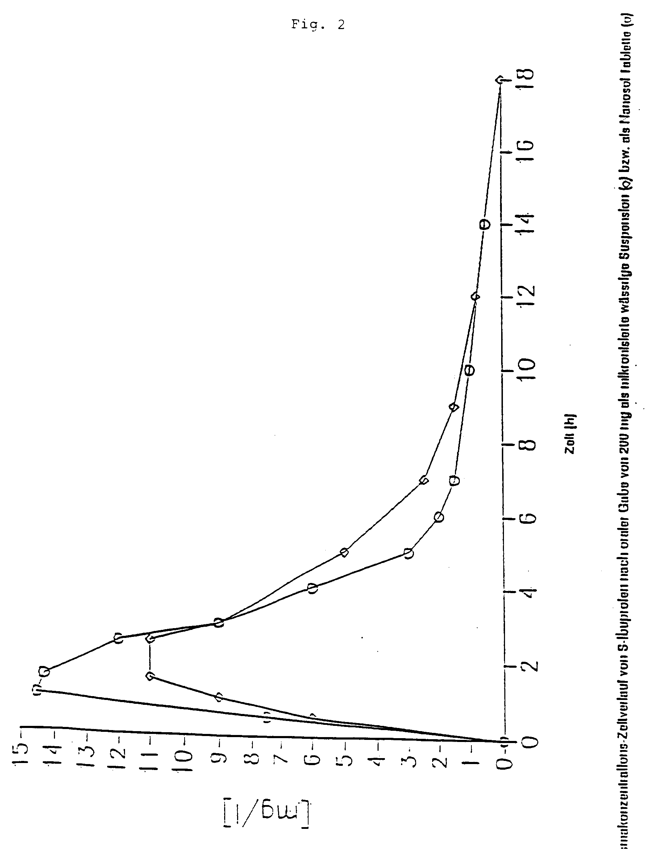

Nach der peroralen Aufnahme eines Arzneistoffs in den Organismus wird seine Resorption, d.h. der Übertritt in den allgemeinen Kreislauf (Biophase) in starkem Maße durch physikalische Barrieren behindert (siehe Abb. 2), nämlich
- durch die Mucus-Schicht und eine wässerige, daran adhärierende Schicht
- die Zellmembranen der intestinalen Epithelzellen mit der daran kovalent gebundenen Glykocalix sowie
- die sogenannten "Tight Junctions", die die Epithelzellen an ihrer apikalen Seite miteinander verbinden.

Diese Barrieren bedingen, daß die Resorption von Arzneistoffen hauptsächlich abhängig von ihrem Verteilungsmechanismus und Ladungszustand- durch die Lipid-Doppelschichten erfolgt (sogenannte passive Diffusion).

Die Epithelzellen des gesamten Magen-Darm-Traktes sind mit einer Mucus-Schicht bedeckt, die aus Mucinen (Glykoproteinen), Elektrolyten, Proteinen und Nucleinsäuren besteht. Vor allem die Glykoproteine bilden mit dem Hauptanteil des Mucus, nämlich Wasser, eine viskose Gelstruktur, die in erster Linie Schutzfunktionen für die darunter liegende Epithelschicht ausübt. Die Mucusschicht ist an die apikale Oberfläche der Epithelzellen über die Glykocalix gebunden. Die Glykocalix hat ebenfalls eine Glykoproteinstruktur, die kovalent an Bausteine der Membran-Doppelschicht der Epithelzellen gebunden ist. Die verzweigten Polysaccharide der Glykocalix, die entweder direkt an amphiphile Moleküle der Doppelmembran oder an die Doppelmembran inkorporierte Proteine kovalent gebunden sind, besitzen geladene N-Acetyl-Neuraminssäure- und Sulfat-Reste und sind daher negativ geladen, was zu einer elektrostatischen Bindung oder Abstoßung von geladenen Arzneistoffmolekülen bzw. von elektrostatisch geladenen Partikeln führen kann. Die Epithelzellmembranen bestehen aus Phospholipid-Doppelschichten, in die Proteine über ihre hydrophoben Bereiche verankert sind. Die Phospholipid-Doppelschichten mit ihrem lipophilen Anteil stellen eine weitere Barriere für den Transport der zu resorbierenden Arzneistoffe dar.

Aus dieser Darstellung geht deutlich hervor, daß geladene Arzneistoffmoleküle bzw. elektrostatisch geladene Partikel daher nur eine sehr geringe Chance haben, über den peroralen Applikationsweg resorbiert zu werden.

Die erfindungsgemäßen Nanosole geben erstmalig die technische Lehre, ein System zu bilden, mit dem diese vorgenannten Resorptionshindernisse zu überwinden sind. Da die Wirkstoff-Nanopartikel durch die Gelatine erfindungsgemäß ladungsneutral stabilisiert werden, kann ihr Transport durch die negativ geladene Glykocalix ohne größere Hindernisse erfolgen, im Gegensatz zu sonstig beschriebenen Nanopartikeln des Standes der Technik, die nicht ladungsneutral stabilisiert werden bzw. stabilisiert werden können. Erfindungsgemäß kann die Einstellung des isoionischen Ladungszustandes zusätzlich noch in Abstimmung auf die physiologischen Verhältnisse erfolgen.

Da die erfindungsgemäßen Wirkstoff-Nanosole die Glykocalix ungehindert passieren können, ohne durch elektrostatische Effekte gebunden bzw. abgestoßen zu werden, erreichen sie damit auch die Oberfläche der Epithelzellen und stehen dort in hoher Konzentration zur Verfügung.

Nun können auch aktive, carriervermittelte Transportmechanismen bzw. Phagozytose einen wesentlichen Beitrag zur Resorption der Wirkstoff-Nanosole liefern.

Die erfindungsgemäß eingesetzten Nanosole zeichnen sich durch hohe Stabilitäten, insbesondere im sauren Bereich aus, ohne zu flocken oder auszukristallisieren. Das bedeutet, daß das Nanosol ausreichend lang während der Magenverweilzeit und unabhängig von auftretenden pH-Schwankungen, z. B. durch Nahrungseinfluß, der Magenmucosa für die Resorption zur Verfügung steht.

Bei pH-Werten unterhalb von 2 kann die Stabilität des Nanosols durch Auswahl einer auf diesen pH-Bereich abgestimmten Gelatinesorte noch verbessert werden (Beispiel 2).

Die Teilchen der Nanosole liegen nach ihrer Herstellung, nach Resuspendierung des getrockneten Pulvers und nach Resuspendierung aus einer Arzneiform in Teilchengrößen von 10 bis 800 nm, bevorzugt unterhalb 400 nm, und darüberhinaus nahezu monodispers vor. Weiterhin ist das Nanosol im resuspendierten Zustand als Nanodispersion im Magen gut verteilt, was optimale Voraussetzungen für die Resorption schafft. Da die Nanopartikel stabilisiert vorliegen, können sie erstaunlicherweise als solche resorbiert werden, ohne daß sie vorher aufgelöst werden müssen. Damit entfällt ein zeitlich vorgelagertes Lösungsgleichgewicht wie bei mikronisierten Pulvern oder wasserlöslichen Salzen in jedem Falle. Sie verhalten sich demnach biopharmazeutisch gesehen wie eine echte Lösung, ohne aber eine solche zu sein.

Somit wird erstmals durch die vorliegende Erfindung eine kontrollierte Resorption im Gastrointestinaltrakt bereits während der Magenverweilzeit möglich. Die Resorption ist nicht mehr auf den Dünndarmbereich beschränkt und es wird eine schnelle Anflutung für Ibuprofen ermöglicht.

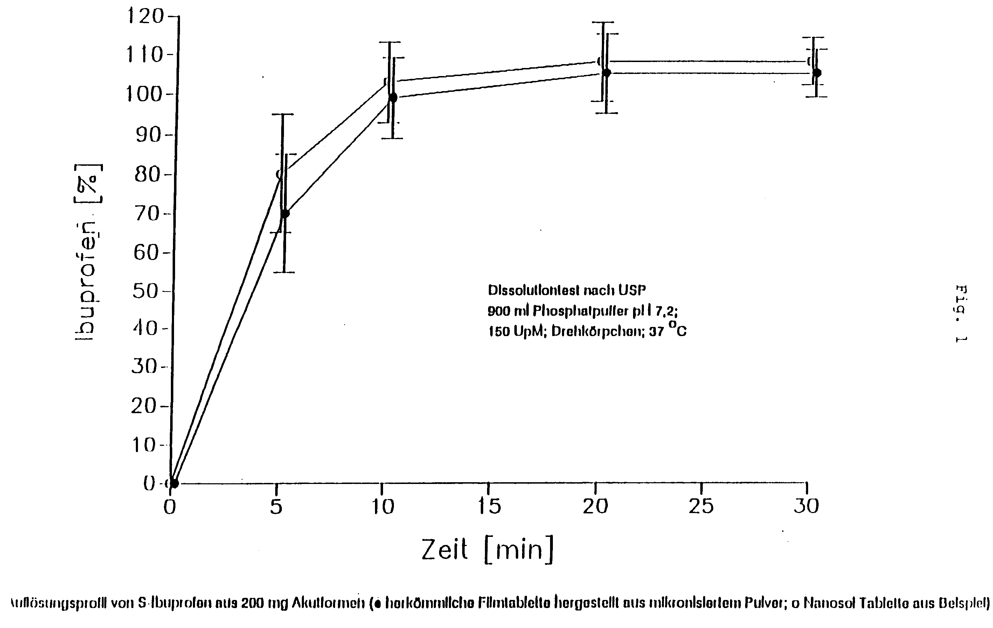

Damit ist es überraschend möglich, gegenüber dem Stand der Technik erstmals mit einer erfindungsgemäß hergestellten Tablette (Fig. 1 und Fig. 2) einen tₘₐₓ-Wert unterhalb von 2 h, insbesondere unterhalb von 1 h zu erreichen.

Zusätzlich läßt sich auch eine Erhöhung des Blutspiegelmaximalwertes cₘₐₓ feststellen (siehe Fig. 2). Die Erhöhung von cₘₐₓ kann daher unter Umständen eine Dosisreduktion bei gleicher Wirksamkeit zur Folge haben. Die schnelle Anflutung führt neben dem schnellen Wirkungseintritt zu einer früheren Elimination aus dem Plasma, sodaß die systemische Belastung gegenüber herkömmlichen Arzneimitteln vorteilhaft verkürzt wird. Die Wirkdauer selbst wird dadurch praktisch nicht verkürzt, weil am Wirkort, insbesondere bei entzündlichen Prozessen, mit einer wesentlich längeren Verweildauer des Wirkstoffs gerechnet werden kann. Die Halbwertszeiten im Plasma betragen im Falle von Ibuprofen ca. 2 h. In der Synovialflüssigkeit wurden dagegen 10 h bis 12 h gefunden.

Wie in-vitro Versuche gezeigt haben, ist aufgrund der aufgeführten langen Stabilitäten der erfindungsgemäßen Nanosole die Gefahr der Rekristallisation im Magen auszuschließen.

Wegen der verschiedenen Wirkungsmechanismen für das S- und das R-Enantiomere (S-Ibuprofen weist in erster Linie periphere Wirkung auf, während das enantiomere R-Ibuprofen vor allem zentral wirkt) können Mischungen aus S- und R-Enantiomeren mit verschiedenen Anteilen der einzelnen Enantiomeren im Einzelfall bevorzugt sein.

Weiterhin kann die Akutform von Ibuprofen auch mit einer Retardformulierung für Ibuprofen kombiniert werden.

Als besondere Ausführungsform kann ein pulverförmiges oder granuliertes Akut-Nanosol mit einer Matrix-Tablette, wie sie in der internationalen (PCT)-Patentanmeldung PCT/DE92/01011 vom 04.12.1992 mit dem Titel "Sol-gesteuerte Thermokolloidmatrix auf Gelatinebasis für perorale Retardformen" der ALFATEC-Pharma GmbH, die der deutschen Patentanmeldung P 4140192.1-41 entspricht, beschrieben wird, z.B. in einer Hartgelatinekapsel kombiniert werden.

Eine solche Arzneiform setzt den Wirkstoff zunächst schnell frei und die Erhaltungsdosis (Matrix-Tablette) mit hoher Reproduzierbarkeit konstant nach einem Geschwindigkeitsgesetz nullter Ordnung.

Das getrocknete Nanosol kann zu Arzneiformen, beispielsweise zu einer Tablette, weiterverarbeitet und daraus resuspendiert werden. Somit wird ein magensaftresistenter Überzug zum Schutz vor "Inaktivierung" des Wirkstoffes durch den sauren Magen-pH überflüssig.

Die Gefahr einer Überdosierung durch Mehrfacheinname wird durch den schnellen Eintritt der Analgesie als Folge der Resorption im Magen ausgeschlossen. Alle genannten Nachteile und Gefahren des magensaftresistenten Überzugs entfallen. Somit dient die vorliegende Erfindung auch der Erhöhung der Patienten-Compliance. Dies alles stellt einen entscheidenden Beitrag zur geforderten Arzneimittelsicherheit dar.

Grundsätzlich läßt sich das erfindungsgemäße Produkt zu allen peroral zu applizierenden Arzneiformen verarbeiten, insbesondere kann es direkt als Pulver in Hartgelatinekapseln abgefüllt werden. Es eignet sich auch hervorragend zur Direkttablettierung. Eine Verarbeitung zu einem Trinkgranulat, schnellauflösenden Pellets oder Trinktabletten ist für die Applikation als schnellanflutende Akutform von besonderem Interesse.

Prinzipiell sind zur Herstellung der erfindungsgemäßen Nanosole, die in dem o.g. deutschen Patent p 4140195 der ALFATEC-Pharma GmbH mit dem Titel "Pharmazeutisch applizierbares Nanosol und Verfahren zu seiner Herstellung" genannten Vorgehensweisen und Verfahrensvarianten geeignet, die im folgenden noch einmal angeführt werden:
Es werden mehrere Verfahren zur Herstellung der Nanosole vorgeschlagen. Dabei handelt es sich um eine beispielhafte, unvollständige Aufzählung. Der Fachmann kann aufgrund seines Fachwissens selbstständig weitere Varianten im Rahmen der vorliegenden Erfindung ausarbeiten:

### Verfahren I

Dieses kann angewendet werden, wenn der Arzneistoff in einer Mischung aus:
einem mit Wasser mischbaren organischen Lösungsmittel und Wasser, oder
aus mehreren mit Wasser mischbaren organischen Lösungsmitteln und Wasser
löslich ist:
a) eine in den Vorversuchen ausgewählte Gelatine wird mit Wasser in Solform überfuhrt;
b) der in den Vorversuchen gefundene pH-Wert der Lösung wird eingestellt;
c) ein oder mehrere mit Wasser mischbare(s), organische(s) Lösungsmittel, vorzugsweise Ethanol, Isopropanol oder Methanol, wird/werden zu dieser Lösung gegeben;
d) der Arzneistoff wird in fester Form zu der Lösung gegeben und gelöst;
e) das/die organische(n) Lösungsmittel wird/werden entfernt, vorzugsweise durch Eindampfen in Vakuum; dabei entsteht das Nanosol;
f) die kolloid-disperse Lösung wird anschließend, vorzugsweise durch Sprüh- oder Gefriertrocknung, getrocknet.

Das organische Lösungsmittel hat die Aufgabe, den Arzneistoff zu lösen und verändert auch die Hydrathülle der Gelatinemoleküle.

### Verfahren II

Diese Ausführungsform kann angewendet werden, wenn der Arzneistoff eine Säure oder eine Base ist, deren Salz in Wasser löslich ist:
a) eine in den Vorversuchen ausgewählte Gelatine wird mit H₂O in die Solform überführt;
b) es wird ein solcher pH-Wert eingestellt, der die Salzbildung des Arzneistoffs ermöglicht;
c) der Arzneistoff wird unter Salzbildung in dem Gelatinesol gelöst;
d) durch Zugabe von Alkohol oder ähnlichen organischen Lösungsmitteln kann die Hydrathülle der Gelatinemoleküle gelockert werden;
e) durch Zugabe einer geeigneten Menge Säure oder Base wird der pH-Wert eingestellt, der zur Bildung des isoionischen Punkts (IIP) führt, dabei entsteht das Nanosol;
f) die kolloid-disperse Lösung wird wie in Verfahren I getrocknet.

Stufe d) ist fakultativ, jedoch bevorzugt.

### Verfahren III

Diese Ausführungsform kann angewendet werden, wenn der Arzneistoff ein Neutralstoff ist:
a) es wird ein Gelatinesol hergestellt, wie unter (1) a) und b) beschrieben.
b) eine zweite Lösung aus einem mit Wasser mischbaren organischen Lösungsmittel, vorzugsweise Ethanol, Methanol, Isopropanol, Aceton und dem Arzneistoff wird hergestellt.
c) die beiden Lösungen werden vereinigt.
d) das organische Lösungsmittel wird entfernt und die kolloid-disperse Lösung wird getrocknet.

### Verfahren IV

a) Wie unter (I) a) und b) beschrieben.
b) In einer zweiten Lösung wird ein kolloid-disperses System mit dem Arzneistoff kurzzeitig gebildet, jedoch ohne Gelatine.
c) Die unter (b) erhaltene Lösung wird kontinuierlich mit der Gelatinelösung vereinigt.

Bei Schritt (IV) c) kann die kontinuierliche Vermischung der unter (IV) a) und b) beschriebenen Lösungen zeitabhängig durch on-line Messung der Teilchengröße mit einem geeigneten Verfahren, wie z.B. durch Laser-Licht-Streuung (BI-FOQELS On-line Particle Sizer), gesteuert werden. Damit ist es möglich, eine gewünschte Partikelgröße kontinuierlich einzustellen.

Alle genannten Verfahren sind auch für Kollagenhydrolysate und Gelatinederivate geeignet und können problemlos in den technischen Maßstab übertragen werden.

Die wesentlichen Schritte können weitgehend automatisiert ablaufen, wobei auch Verfahren I bis III kontinuierlich durchführbar sind. Im Falle der Akutform für 2-Arylpropionsäurederivate seien als bevorzugt geeignete Verfahren die Varianten Nr. II und III genannt.

Für die erfindungsgemäßen Akutformen eignen sich alle Gelatinen, Gelatinederivate, Kollagenhydrolysate und fraktionierte Gelatine, sowie deren Mischungen. Gelatinesorten, die einen erfindungsgemäß beschriebenen isoelektrischen Punkt (IEP) aufweisen, der nicht handelsüblich ist, können nach den Beispielen I bis III aus o.g. deutscher Patentanmeldung hergestellt werden.

Gegenüber handelsüblichen Produkten führt die Verwendung von Gelatine, die auf spezielle Weise hergestellt wurde, zu erfindungsgemäß beschriebenen Nanosolen mit erhöhter Stabilität.

Beispiele für die Herstellung erfindungsgemäß besonders geeigneter Gelatinequalitäten werden unten gegeben.

Beispiele für die Herstellung von erfindungsgemäß besonders geeigneten Gelatinesorten mit isoelektrischen Punkten von 3,5 bis 9,5

### Beispiel I:

### Verfahren zur Erzielung eines IEP's von 7,5 bis 9,5

Kollagenhaltiges Ausgangsmaterial wie z.B. Schweineschwarten werden mit einer wäßrigen Lösung einer 0,45 N Mineralsäure, vorzugsweise Schwefelsäure, im Flottenverhältnis 1:1 12 bis 20 Stunden behandelt. Anschließend wird der Säureüberschuß durch mehrmaliges Waschen entfernt, wobei zur Abkürzung des Verfahrens Natriumhydrogencarbonat verwendet werden kann. Die Extraktion des sudreifen Materials erfolgt mit heißem Wasser bei 55 - 80° C bei einem pH von 2,5 bis 4,5. Bei pH-Werten unterhalb von 3,5 kann ein IEP von 8,5 bis 9,5 erreicht werden, bei pH-Werten oberhalb 3,5 liegt der IEP bei 7 bis 8,5. Auf diese Weise lassen sich verschiedene IEP's von 7 bis 9,5 in direkter Abhängigkeit vom pH-Wert während der Extraktion erzielen.

Nach der Verfahrensstufe der Extraktion wird die wäßrige Lösung neutralisiert und wie üblich aufgearbeitet.

Durch dieses Verfahren kann man weiterhin in Abhängigkeit von der gewählten Temperatur während der Extraktion Gelatinesorten mit hohen bis mittleren Molekulargewichtsverteilungen erhalten.

Bei Temperaturen von 50-55° C erhält man besonders hochviskose und hochbloomige Qualitäten. Gelatinesorten mit niedrigem Molekulargewicht bzw. kaltwasserlösliche Gelatinen können durch gezielten Abbau mit Kollagenasen erhalten werden.

### Beispiel II:

### Verfahren zur Erzielung eines IEP's von 4 bis 7,5

Das kollagenhaltige Ausgangsmaterial wird zur Entfernung von Fremdstoffen zunächst gewaschen, zerkleinert und anschließend durch Zusatz von Magnesit, Natronlauge oder Calciumhydroxid durch gründliches Vermischen im Flottenverhältnis 1:1,2 homogen alkalisch gemacht. Das so vorbehandelte Material wird kurzzeitig druckhydrolytisch bei 1,01 x 10⁵ bis 2,02 x 10⁵ Pa und einem pH-Wert der wäßrigen Lösung von 8-14 aufgeschlossen. Nach dem Aufschluß wird sofort neutralisiert und die noch heiße wäßrige Gelatinelösung wie üblich filtriert, entsalzt, aufkonzentriert und getrocknet.

Nimmt man ein schwach basisches Aufschlußmittel wie Magnesit, erhält man einen IEP von 6 bis 7,5, sofern man bei 1,01 x 10⁵ Pa arbeitet. IEP's von 5 bis 6 erhält man bei Einsatz einer verdünnten Kalkmilchsuspension und bei Verwendung von 0,005 bis 0,1 N Natronlauge können IEP's von 4 bis 5 erzielt werden.

Gelatinesorten mit geringem Racemisierungsgrad und niedrigem Peptidanteil lassen sich bei Druckverhältnissen von 1,01 x 10⁵ Pa und Verweilzeiten von maximal 10 Min. erreichen.

Mittel- bis niedrigmolekulare bis hin zu kaltwasserlöslichen Sorten ergeben sich durch entsprechend längere Verweilzeiten.

### Beispiel III:

### Verfahren zur Erzielung eines IEP's von 3,5 bis 6

Kollagenhaltiges Ausgangsmaterial, vorzugsweise Spalt bzw. Ossein, wird nach der Eingangswäsche einem Kurzzeitäscher unterworfen. Hierbei bieten sich zwei Verfahrensvarianten im Flottenverhältnis 1:1,3 an, die entweder eine gesättigte Kalkmilchsuspension oder eine 0,1 bis 1 N Natronlauge zum Einsatz bringen.

Bei Verwendung einer Kalkmilchsuspension wird das Rohmaterial unter ständiger Bewegung maximal 3 bis 4 Wochen aufgeschlossen. Anschließend wird das Material durch Säurezugabe neutralisiert und mehrmals gewaschen. Die weitere Aufarbeitung folgt wie üblich. Auf diese Weise lassen sich IEP's von 4 bis 6 einstellen.

Bei Einsatz von Natronlauge läßt sich der Äscherprozeß nochmals verkürzen, wobei bei Konzentrationen von 1 N Natronlauge das Material je nach Zerkleinerungsgrad bereits nach 6 - 12 Stunden aufgeschlossen ist. Die Neutralisation erfolgt mit äquimolaren Mengen Mineralsäure und die Neutralsalze werden durch mehrmaliges Waschen oder durch Entsalzen der in der Extraktion gewonnenen wäßrigen Gelatinelösung entfernt. Bei dieser Verfahrensvariante lassen sich IEP's von 3,5 bis 5 erhalten.

Besonders peptidarme Gelatinesorten werden bei kurzer Verweilzeit im Äscher erhalten. Man kann so Gelatinesorten mit hoher bis mittlerer Molekulargewichtsverteilung (M = 10⁴ - 10⁷ D) erhalten.

Niedrigmolekulare bis kaltwasserlösliche Gelatinesorten kann man durch thermischen Abbau bzw. enzymatisch erhalten.

Bevorzugt werden im Falle der 2-Arylpropionsäurederivate Gelatinesorten mit IEP von 3,5 bis 9,5 eingesetzt.

Übliche pharmazeutische Hilfsstoffe und/oder weitere Makromoleküle können, sofern sie technologisch erforderlich sind, in flüssigem oder getrocknetem Zustand den erfindungsgemäßen Nanosolen zugesetzt werden.

Zum Beispiel kann ein Zusatz von Polyvinylpyrrolidon im Mengenverhältnis Gelatine zu Polyvinylpyrrolidon im Bereich von 5:1 bis 500:1 geeignet sein.

Eine Akutform im Sinne der Erfindung, die z.B. zu Tabletten verarbeitet wird oder lyophilisiert werden soll, kann durch Zusatz von niedrigmolekularen Polyvinylpyrrolidonsorten im Bereich von 10:1 bis 50:1 in den technologischen Verarbeitungseigenschaften verbessert werden, ohne daß die Stabilität der Nanosole negativ beeinflußt wird.

Die in den folgenden Beispielen bevorzugten Herstellungsverfahren, Vorgehensweisen und Bezeichnungen beziehen sich wie folgt auf die deutschen Patentanmeldungen "Pharmazeutisch applizierbares Nanosol und Verfahren zu seiner Herstellung" (P 41 40 195.6) bzw. die oben genannten Verfahren und Beispiele:
Nanosol-Herstellung: Verfahren II und III
Gelatineherstellung: Beispiel I bis III
Vortest: siehe folgende Beschreibung:

### Vortest:

Wie eingangs schon erwähnt und wie aus Fig.1 ersichtlich ist, hängt die absolute, maximal mögliche Nettoladung eines einzelnen Gelatinemoleküls hauptsächlich von der Anzahl der freien COOH- und NH₂-Gruppen und dem pH-Wert der Lösung ab. Da sich Typ A, B, Kollagenhydrolysate oder Gelatinederivate in der Anzahl freier COOH-Gruppen unterscheiden, ist damit auch ihre maximal mögliche Nettoladung unterschiedlich. Bei Gelatinederivaten kann der Ladungszustand zusätzlich von der Art der Modifizierung abhängen.

Bei der Durchführung des erfindungsgemäßen Verfahrens wählt man in einem Vortest die geeignete Gelatine und den geeigneten pH-Wert aus.

Zunächst wird ein den physikalisch-chemischen Eigenschaften des Arzneistoffs angepaßter Arbeits-pH-Bereich gewählt. Als physikalisch-chemische Eigenschaft des Arzneistoffs sind vor allem zu berücksichtigen: Die Löslichkeit (in organischen Lösungsmitteln bzw. Wasser), seine Eigenschaft als Säure, Base oder Neutralstoff sowie seine Stabilität gegenüber Säuren und Laugen.

In einem ersten Schnelltest wird festgestellt, welche Ladung die ausgefällten Partikel besitzen. Daraus ergibt sich, unter Berücksichtigung des Arbeits-pH-Bereichs, die Wahl eines geeigneten Gelatinetyps. Sind die Teilchen beispielsweise negativ geladen, sucht man eine Gelatine aus, die unter den gegebenen pH-Bedingungen positiv geladen ist. Dieser Schnelltest zur Feststellung der Partikelladung hat die Vorteile, daß er ohne großen apparativen und zeitlichen Aufwand durchgeführt werden kann. So kann auf eine zeitaufwendige und ungenaue Zeta-Potential-Messung gänzlich verzichtet werden.

In vielen Fällen wird es ausreichend sein, für diesen Schnelltest zwei handelsübliche Gelatinen Typ A und B mit einem IEP von 9,5 bzw. 3,5 mit Peptidanteilen <30 % und einer Bloomzahl von 200, die weiterhin als Standardgelatinen bezeichnet werden, bei einem pH-wert von 6 in die Solform zu überführen (5%ige wäßrige Lösung) und den Arzneistoff in einem mit Wasser mischbaren Lösungsmittel, wie z. B. Ethanol, Isopropanol oder Aceton, zu lösen und jeweils mit den Gelatinelösungen homogen zu mischen. Bei gleicher Dosierung des Arzneistoffs wird sich bei der in ihrem Ladungszustand nicht geeigneten Gelatine ein kolloidales System entweder nicht ausbilden oder sofort instabil werden bzw. der Arzneistoff ausflocken. Sind die entstehenden Partikel negativ geladen, werden sie eher von Gelatinelösung mit Typ A, der bei einem pH-Wert von 6 positiv geladen ist, stabilisiert als von der Lösung mit Gelatine Typ B; im Gegenteil wird in diesem Fall Typ B entweder kein kolloidales System ausbilden oder das System sofort destabilisieren. Das Ausflocken der Teilchen läßt sich z. B. über eine einfache Trübungs-Messung verfolgen.

Bei diesem Schnelltest muß auf jeden Fall der Arbeits-pH-Bereich beachtet werden. Man kann auch andere Gelatinen als Standard auswählen, sie müssen jedoch in ihrem IEP so gewählt werden, daß sie bei diesem pH-Wert entgegengesetzte Nettoladung tragen (siehe auch Fig.1). In den meisten Fällen werden die besagten Standardgelatinen Typ A und B für diesen Schnelltest ausreichen.

Ausgehend vom Ergebnis des Vorversuchs werden nun durch schrittweise Variation des IEP's durch Verwendung entsprechender Gelatinesorten und des pH-Wertes der Lösung in kleineren Bereichen (z. B. 0,1 pH-Schritte) die optimalen Bedingungen zur Bildung der Nanosole ermittelt. D.h. es muß das Stabilitätsoptimum, das durch den isoionischen Punkt (IIP) gekennzeichnet ist, gefunden werden, um eine ausreichende Stabilität für die genannten pharmazeutischen Anwendungen zu gewährleisten.

Es kann durchaus der Fall sein, daß eine im Sinne der Erfindung akzeptable Stabilität der Nanosole bereits in einem engeren pH-Bereich (ca. 0,5 Einheiten) um den isoionischen Punkt gefunden wird, so daß eine Einstellung dieses Punktes selbst nicht unbedingt notwendig ist. Andererseits können auch mehrere Gelatinen zu den gleichen, stabilen Ergebnissen führen. So kann beispielsweise (Beispiel 5) mit dem oralen Antidiabetikum Glibenclamid bei einem Gelatinetyp B mit einem IEP von 5,5 das Stabilitätsoptimum bei einem pH-Wert von 3,2 liegen, während bei einem Gelatinetyp B mit einem IEP von 3,8 das Stabilitätsoptimum bei einem pH-Wert von 2,2 liegt.

Gekennzeichnet durch ein Stabilitätsmaximum, wurde in beiden Fällen der isoionische Punkt erreicht (die Abhängigkeit der Nettoladung vom pH-Wert und dem IEP muß nicht linear sein, da sie durch den pKₛ-Wert der vorhandenen COOH- bzw. NH₃⁺ - Gruppen gegeben ist).

Gelatine ist ein aus kollagenhaltigem Material gewonnenes Skleroprotein, das je nach Herstellungsprozeß unterschiedliche Eigenschaften hat. Es existieren Molekulargewichtsbereiche von einigen Tausend D bis hin zu einigen Millionen D, die in ihrer Molekulargewichtszusammensetzung und in ihrem physikalisch-chemischen Verhalten höchst unterschiedlich sein können. Bei genauer Kenntnis dieser Zusammenhänge lassen sich neue pharmazeutische Anwendungen finden, die sich durch hohe Reproduzierbarkeit und einfache technologische Verarbeitung auszeichnen. Einzelheiten können aus den o.g. Anmeldungen entnommen werden. Bei besonders schonender Herstellungsweise kann man Gelatinesorten erhalten, die nur einen geringen Anteil an rechtsdrehenden Aminosäuren aufweisen und somit ähnlich aufgebaut sind wie das native Kollagenmolekül. Diese Gelatinen zeichnen sich zum Beispiel durch besonders gute Stabilisierungseigenschaften für Nanosole aus. Eine solche Gelatine ist erfindungsgemäß vorteilhaft geeignet. Je nach Aufarbeitung des Rohmaterials (saurer oder basischer Aufschluß) erhält man Gelatinen, deren isoelektrische Punkte ganz unterschiedlich sind. Durch spezielle Herstellungstechniken können isoelektrische Punkte gezielt hergestellt werden, wobei die Molekulargewichtsverteilung auf den Anwendungsfall abgestimmt sein kann.

In Abhängigkeit von der Herstellungsweise von Gelatine (Ausmaß des Abbaus des nativen Kollagens und saures bzw. alkalisches Aufschlußverfahren) weist Gelatine vom Typ A oder Typ B ein charakteristisches Molekulargewichtsspektrum bzw. Molekulargewichtsverteilung auf. In Tabelle 1 sind die Molekulargewichtsverteilungen von verschiedenen Gelatinetypen bzw. von Kollagenhydrolysaten angegeben, sowie der prozentuale Anteil (Häufigkeit) einzelner Molekulargewichtsbereiche.

**Tabelle 1**

| Molekulargewichtsverteilung von verschiedenen bekannten Gelatinetypen bzw. von bekannten Kollagenhydrolysaten | | | | | | | |
|---|---|---|---|---|---|---|---|
| Molekulargewichtsverteilung (KD) | Natives Kollagen % | Gelatine Typ B % | Gelatine Typ A % | Kollagenhydrolysat Gelita®-Collagel A | Kollagenhydrolysat Gelita®-Collagel B | Kollagenhydrolysat Gelita®-Sol C | Elastinhydrolysat Gelita®-Gelastin |
| > 360 | 100 | 18,0 | 18,0 | 0 | 0 | 0 | 0 |
| 285 | 0 | 7,0 | 9,0 | 0 | 0 | 0 | 0 |
| 145-237 | 0 | 20,0 | 34,0 | 1,0 | 1,5 | 0 | 0 |
| 95 | 0 | 26,0 | 11,0 | 0 | 0 | 0 | 0 |
| 95-50 | 0 | 16,3 | 13,4 | 2,6 | 4,0 | 1,1 | 0 |
| 50-20 | 0 | 7,4 | 9,1 | 18,0 | 14,5 | 0,3 | 0 |
| 20-10 | 0 | 3,9 | 3,8 | 43,0 | 31,5 | 3,7 | 0,2 |
| 10-5 | 0 | 3,0 | 3,0 | 15,4 | 20,0 | 12,2 | 5,2 |
| 5-2 | 0 | 0 | 0 | 6,0 | 14,0 | 26,0 | 93,9 |
| 2-1 | 0 | 0 | 0 | 7,0 | 8,0 | 23,0 | 0 |
| <1 | 0 | 0 | 0 | 6,5 | 7,0 | 34,0 | 0 |
| MG | 360 | 165 | 185 | 12 - 18 | 12 - 18 | 3 | 2-3 |

Man erkennt in den einzelnen Spalten deutlich das Überwiegen eines einzelnen Bereiches im Vergleich zu den übrigen Molekulargewichtsbereichen derselben Gelatine. Dieser Bereich stellt also das Maximum der Molekulargewichtsverteilung dar (es liegt z.B. bei der in der Abbildung aufgeführten Gelatine Typ B bei 95 kD). Der Begriff des "Maximums der Molekulargewichtsverteilung" ist jedoch streng zu trennen von dem Begriff des "durchschnittlichen mittleren Molekulargewichts". Dieser Mittelwert liegt bei der erwähnten Gelatine vom Typ B bei 165 kD.

Im Falle von S-Ibuprofen, insbesondere bei höherer Dosierung sind bevorzugt Gelatinesorten geeignet, die einen Anteil an rechtsdrehenden Aminosäuren unterhalb von 20% aufweisen und deren Maximum der Molekulargewichtsverteilung unterhalb 10⁵ D liegt. Zur Tablettenherstellung, wie sie üblicherweise bei Schmerzmitteln im Vordergrund steht, eignen sich bevorzugt Gelatinesorten mit Bloomwerten von 0 - 50. Bei den genannten Gelatinen kann vorteilhafterweise ein Gewichtsverhältnis Gelatine zu Wirkstoff von 0,5:1 bis 3:1 eingehalten werden.

Bei der Formulierung von Akut- bzw. Retardpräparaten macht der Pharmazeut einen grundsätzlichen Unterschied zwischen:
1. galenischer Zubereitung, d.h. einer Freisetzung des Arzneistoffes, z.B. aus einer Tablette in zeitlich schneller (Akutform) oder verlangsamter (Retardform) Weise;
und
2. dem arzneistoffspezifischen Resorptionsort, wie z.B. Magen oder bestimmte Darmabschnitte.

Die erfindungsgemäßen Nanosole sind in der Lage, unabhängig von der galenischen Zubereitung, aufgrund ihrer speziellen Zusammensetzung, im gesamten gastrointestinalen Bereich resorbiert zu werden. Sie können daher vorteilhaft zu Akut- bzw. Retardarzneiformen weiterverarbeitet werden.

Für die erfindungsgemäß verwendeten Nanosole eignen sich auch handelsübliche Gelatinen, fraktionierte Gelatine, Kollagenhydrolysate und Gelatinederivate, insbesondere solche Sorten, die durch eine niedrige Bloomzahl von 0 (kaltwasserlösliche Gelatine oder Kollagenhydrolysate) bis 240 Bloom, vorzugsweise 0 bis 170 Bloom charakterisiert sind. Im Falle des Ibuprofens werden bevorzugt Gelatinesorten mit IEP's von 3,5 bis 7,5 eingesetzt

Für die Sprüh- oder Gefriertrocknung von S-Ibuprofen-Nanosolen hat sich ein Zusatz von Polvinylpyrrolidon (PVP) zur wäßrigen Gelatinelösung, insbesondere PVP K 15 oder PVP K 25 im Gewichtsverhältnis von 1:5 bis 1:30 als vorteilhaft gezeigt, wobei ohne negative Beeinflussung der Stabilität des Nanosols ein gut rieselfähiges Pulver erhalten wird.

### Beispiel 1:

- Wirkstoff:: S-Ibuprofen, enantiomerreine Wirkstoffsäure
- Gelatinetyp:: handelsüblich, Typ B, 40 Bloom
- Nanosol-Herstellung:: analog Verfahren II
- Gewichtsverhältnis Gelatine/Wirkstoff:: 2:1
Der Arbeits-pH-Bereich für S-Ibuprofen liegt unterhalb seines pKₛ-Wertes von 4,6.

Nach Durchführung des erfindungsgemäßen Vortests und der Meßreihe zur Bestimmung der optimalen Gelatinesorte wird ein Stabilitätsmaximum mit einer Gelatine Typ B (IEP 4,9) bei einem pH-Wert von 3,0 ermittelt.

600 g oben genannter Gelatine werden in 10 l Wasser gelöst. 300 g S-Ibuprofen werden in 0,8 l Natronlauge (10%ig) gelöst und zur Gelatinelösung gegeben. Es wird so lange gerührt, bis eine völlig klare Lösung entsteht. Danach wird durch Zugabe von Salzsäure auf pH 3.0 eingestellt, wobei sich das Nanosol bildet.

Die Nanosol-Lösung wird wie üblich aufkonzentriert und sprühgetrocknet. Das getrocknete Nanosol wird zu schnell auflösenden Tablette mit jeweils 100 mg S-Ibuprofengehalt verarbeitet.

Unter in-vitro-Bedingungen (900 ml 0,1 N HCl, paddle, 100 rpm, 37°C) setzt ein Teilchenwachstum nach 12 Stunden ein.

### Beispiel 2:

Man stellt ein S-Ibuprofen-Nanosol wie in Beispiel 1 her, wählt jedoch eine Gelatine mit einem IEP von 3,9 aus, die bei einem pH-Wert von 2 ein Stabilitätsoptimum aufweist.

Der wie in Beispiel 1 durchgeführte in vitro-Test unter gleichen Prüfbedingungen ergibt eine um 30% erhöhte Stabilität des Nanosols.

### Beispiel 3:

Analog Beispiel 1, nur wird die Gelatine vor der Herstellung des Gelatinelösung mit 20 g Polyvinylpyrrolidon (PVP K 15) gemischt.
Das erhaltene Nanosol wird sprühgetrocknet und direkt zur Tablette verpreßt.

In einem Dissolutiontest nach USP (900 ml Phosphatpuffer pH7,2, 150 rpm, Drehkörbchen, 37°C) wird eine Auflösezeit der Tabletten von unter 15 Minuten ermittelt. Im Vergleich dazu werden Tabletten aus der Charge nach Beispiel 1 unter diesen Bedingungen untersucht und zeigen im Durchschnitt um 20% höhere Auflösezeiten.

### Beispiel 4:

### S-Ibuprofen-Trinkgranulat

- Wirkstoff:: S-Ibuprofen, enantiomerreine Wirkstoffsäure
- Gelatinetyp:: Typ B, kaltwasserlöslich, Herstellung Beispiel II
- Nanosol-Herstellung:: analog Verfahren III
- Gewichtsverhältnis Gelatine/Wirkstoff:: 10:1
Das Stabilitätsoptimum nach Vortest und Meßreihe zeigt eine Gelatine mit einem IEP von 4,9 bei einem pH-Wert von 3.

300 g obiger Gelatinesorte werden in 5 l Wasser gelöst und mit Salzsäure auf einen pH-Wert von 3 eingestellt. 30 g S-Ibuprofen werden in 0,25 l Alkohol gelöst. Beide Lösungen werden vereinigt, das organische Lösungsmittel unter Vakuum entfernt und das Nanosol wie üblich aufkonzentriert und sprühgetrocknet. Das erhaltene Pulver wird granuliert und mit einem Gehalt von 100 mg S-Ibuprofen pro Einzelgranulat abgefüllt. Das Granulat löst sich in Wasser von 20°C innerhalb von 3 Minuten auf.

### Beispiel 5:

- Wirkstoff:: S-Ibuprofen, enantiomerreine Wirkstoffsäure
- Gelatinetyp:: Gelatine mit Peptidanteil 90%,Typ B, Herstellung Beispiel III
- Nanosol-Herstellung:: analog Verfahren II
- Gewichtsverhältnis Gelatine/Wirkstoff:: 1:1
Es werden Tabletten entsprechend Beispiel 1 hergestellt, wobei eine Dosierung von 200 mg S-Ibuprofen pro Tablette gewählt wird.

Das Auflösungsprofil (siehe Fig. 1) ergibt gegenüber herkömmlichen Filmtabletten einen nahezu identischen Kurvenverlauf.

Der Plasmakonzentrations-Zeitverlauf (siehe Fig. 2), zeigt, daß die gleiche Nanosol-Tablette einen Plasmakonzentrations-Zeitverlauf erreicht, der sowohl hinsichtlich tₘₐₓ als auch cₘₐₓ selbst einer mikronisierten, wäßrigen Suspension nach oraler Gabe deutlich überlegen ist.

## Patentansprüche

1. Akut-Arzneimittel zur Behandlung von schmerzhaften und/oder entzündlichen sowie fieberhaften Erkrankungen, enthaltend Ibuprofen neben üblichen pharmazeutischen Trägern und Hilfsstoffen, dadurch gekennzeichnet, daß das Ibuprofen in Form eines pharmazeutisch applizierbaren Nanosols vorliegt, das als Träger im wesentlichen Gelatine, ein Kollagenhydrolysat oder ein Gelatinederivat enthält, wobei das Nanosol
a) eine innere Phase aus dem Ibuprofen, das eine Teilchengröße von 10 - 800 nm aufweist und eine Oberflächenladung besitzt,
b) eine äußere Phase aus Gelatine, einem Kollagenhydrolysat oder einem Gelatinederivat, welche(s) gegensinnig geladen ist, und
c) einen annähernd oder vollständigen isoionischen Ladungszustand der inneren und äußeren Phase
aufweist und
d) physiologisch resorbierbar ist.

2. Arzneimittel nach Anspruch 1, dadurch gekennzeichnet, daß das Ibuprofen als flüssige, wäßrige Nanodispersion vorliegt.

3. Arzneimittel nach Anspruch 1, dadurch gekennzeichnet, daß das Ibuprofen als feste, resuspendierbare Nanodispersion vorliegt.

4. Arzneimittel nach einem der Ansprüche 1 bis 3, dadurch gekennzeichnet, daß das Ibuprofen eine durchschnittliche Teilchengröße unterhalb von 400 nm aufweist.

5. Arzneimittel nach einem der Ansprüche 1 bis 4, dadurch gekennzeichnet, daß die Gelatine ein Maximum der Molekulargewichtsverteilung unterhalb von 10⁵ D und Bloomwerte von 0 - 240 aufweist.

6. Arzneimittel nach einem der Ansprüche 1 bis 5, dadurch gekennzeichnet, daß die Gelatine einen Bloomwert von 0 - 170 aufweist.

7. Arzneimittel nach Anspruch 6, dadurch gekennzeichnet, daß die Gelatine einen Peptidanteil von 50 - 90% aufweist.

8. Arzneimittel nach einem der Ansprüche 1 bis 6, dadurch gekennzeichnet, daß die Gelatine einen Anteil an rechtsdrehenden Aminosäuren unterhalb von 20% aufweist.

9. Arzneimittel nach einem der Ansprüche 1 bis 8, dadurch gekennzeichnet, daß das Gewichtsverhältnis Gelatine zu Wirkstoff 0,5:1 bis 10:1, bevorzugt 0,5:1 bis 3:1 ist.

10. Arzneimittel nach einem der Ansprüche 1 bis 9, gekennzeichnet durch eine äußere Phase des Nanosols, die zusätzlich Polyvinylpyrrolidon in einem Gewichtsverhältnis von Gelatine zu Polyvinylpyrrolidon wie 5:1 bis 500:1 enthält, insbesondere von 5:1 bis 30:1.

11. Arzneimittel nach einem der Ansprüche 1 bis 10, dadurch gekennzeichnet, daß die Arzneiform eine schnell auflösende Tablette ist.

12. Arzneimittel nach einem der Ansprüche 1 bis 10, dadurch gekennzeichnet, daß die Arzneiform ein schnell auflösendes Pulver oder Granulat ist.

13. Arzneimittel zur Behandlung von schmerzhaften und/oder entzündlichen, sowie fieberhaften Erkrankungen, enthaltend Ibuprofen neben üblichen pharmazeutischen Trägern und Hilfsstoffen, dadurch gekennzeichnet, daß es teilweise als Akutform, teilweise als Retardform mit Ibuprofen in Form eines pharmazeutisch applizierbaren Nanosols vorliegt, wobei das Nanosol
a) eine innere Phase aus dem Ibuprofen, das eine Teilchengröße von 10 - 800 nm aufweist und eine Oberflächenladung besitzt,
b) eine äußere Phase aus Gelatine, einem Kollagenhydrolysat oder einem Gelatinederivat, welche(s) gegensinnig geladen ist, und
c) einen annähernd oder vollständigen isoionischen Ladungszustand der inneren und äußeren Phase
aufweist und
d) physiologisch resorbierbar ist.

14. Arzneimittel nach Anspruch 13, dadurch gekennzeichnet, daß die Arzneiform eine Hartgelatinekapsel ist, die ein schnell auflösendes Pulver mit einer langsam auflösenden Tablette enthält.

15. Arzneimittel nach einem der Ansprüche 1 bis 14, dadurch gekennzeichnet, daß das Ibuprofen als Racemat vorliegt.

16. Arzneimittel nach einem der Ansprüche 1 bis 14, dadurch gekennzeichnet, daß das Ibuprofen als Pseudoracemat oder als Gemisch aus dem Racemat mit seinen Enantiomeren vorliegt.

17. Arzneimittel nach einem der Ansprüche 1 bis 14, dadurch gekennzeichnet, daß das Ibuprofen als reines S- oder R-Ibuprofen vorliegt.

18. Arzneimittel nach einem der Ansprüche 1 bis 14, dadurch gekennzeichnet, daß das Ibuprofen als Gemisch von unterschiedlichen Anteilen an S- und R-Enantiomeren vorliegt.

19. Verwendung eines pharmazeutisch applizierbaren Nanosols von Ibuprofen, das zu mindestens 50% als R-Ibuprofen vorliegt, das
a) eine innere Phase aus dem Ibuprofen, das eine Teilchengröße von 10 - 800 nm aufweist und eine Oberflächenladung besitzt,
b) eine äußere Phase aus Gelatine, einem Kollagenhydrolysat oder einem Gelatinederivat, welche(s) gegensinnig geladen ist, und
c) einen annähernd oder vollständigen isoionischen Ladungszustand der inneren und äußeren Phase
aufweist und
d) physiologisch resorbierbar ist,
zur Herstellung von Arzneimitteln mit akuter analgetischer und/oder antirheumatischer, sowie antipyretischer Wirkung.

20. Verwendung eines pharmazeutisch applizierbaren Nanosols von Ibuprofen, das zu mehr als 50% als S-Ibuprofen vorliegt, das
a) eine innere Phase aus dem Ibuprofen, das eine Teilchengröße von 10 - 800 nm aufweist und eine Oberflächenladung besitzt,
b) eine äußere Phase aus Gelatine, einem Kollagenhydrolysat oder einem Gelatinederivat, welche(s) gegensinnig geladen ist, und
c) einen annähernd oder vollständigen isoionischen Ladungszustand der inneren und äußeren Phase
aufweist und
d) physiologisch resorbierbar ist,
zur Herstellung von Arneimitteln mit akuter analgetischer und/oder antirheumatischer, sowie antipyretischer Wirkung.

21. Verwendung eines pharmazeutisch applizierbaren Nanosols von Ibuprofen, das zu mindestens 50% als S- oder R-Ibuprofen vorliegt, das
a) eine innere Phase aus dem Ibuprofen, das eine Teilchengröße von 10 - 800 nm aufweist und eine Oberflächenladung besitzt,
b) eine äußere Phase aus Gelatine, einem Kollagenhydrolysat oder einem Gelatinederivat, welche(s) gegensinnig geladen ist, und
c) einen annähernd oder vollständigen isoionischen Ladungszustand der inneren und äußeren Phase aufweist und
d) physiologisch resorbierbar ist,
zur Herstellung von Arzneimitteln mit analgetischer und/oder antirheumatischer Akut- und Retardwirkung.

## Claims

1. Immediate-effect medicament for the treatment of painful and/or inflammatory, and also febrile, disorders, containing ibuprofen in addition to customary pharmaceutical excipients and auxiliaries, characterized in that the ibuprofen is present in the form of a pharmaceutically administrable nanosol which essentially contains gelatin, a collagen hydrolysate or a gelatin derivative as the excipient, the nanosol
a) having an inner phase of ibuprofen which has a particle size of 10 - 800 nm and possesses a surface charge,
b) an outer phase of gelatin, a collagen hydrolysate or a gelatin derivative, which is oppositely charged, and
c) an approximate or complete isoionic state of charge of the inner and outer phase and
d) being physiologically absorbable.

2. Medicament according to Claim 1, characterized in that the ibuprofen is present as a liquid, aqueous nanodispersion.

3. Medicament according to Claim 1, characterized in that the ibuprofen is present as a solid, resuspensible nanodispersion.

4. Medicament according to one of Claims 1 to 3, characterized in that the ibuprofen has an average particle size of below 400 nm.

5. Medicament according to one of Claims 1 to 4, characterized in that the gelatin has a maximum in the molecular weight distribution of below 10⁵ D and bloom values of 0 - 240.

6. Medicament according to one of Claims 1 to 5, characterized in that the gelatin has a bloom value of 0 - 170.

7. Medicament according to Claim 6, characterized in that the gelatin has a peptide content of 50 - 90 %.

8. Medicament according to one of Claims 1 to 6, characterized in that the gelatin has a content of dextrorotatory amino acids of below 20 %.

9. Medicament according to one of Claims 1 to 8, characterized in that the weight ratio of gelatin to active compound is 0.5 : 1 to 10 : 1, preferably 0.5 : 1 to 3 : 1.

10. Medicament according to one of Claims 1 to 9, characterized by an outer phase of the nanosol which additionally contains polyvinylpyrrolidone in a weight ratio of gelatin to polyvinylpyrrolidone such as 5 : 1 to 500 : 1, in particular 5 : 1 to 30 : 1.

11. Medicament according to one of Claims 1 to 10, characterized in that the pharmaceutical form is a rapidly dissolving tablet.

12. Medicament according to one of Claims 1 to 10, characterized in that the pharmaceutical form is a rapidly dissolving powder or granules.

13. Medicament for the treatment of painful and/or inflammatory, and also febrile, disorders, containing ibuprofen in addition to customary pharmaceutical excipients and auxiliaries, characterized in that it is partly present as an immediate-effect form and partly as a sustained-release form containing ibuprofen in the form of a pharmaceutically administrable nanosol, the nanosol
a) having an inner phase of ibuprofen which has a particle size of 10 - 800 nm and possesses a surface charge,
b) an outer phase of gelatin, a collagen hydrolysate or a gelatin derivative, which is oppositely charged, and
c) an approximate or complete isoionic state of charge of the inner and outer phase and
d) being physiologically absorbable.

14. Medicament according to Claim 13, characterized in that the pharmaceutical form is a hard gelatin capsule containing a rapidly dissolving powder with a slowly dissolving tablet.

15. Medicament according to one of Claims 1 to 14, characterized in that the ibuprofen is present as a racemate.

16. Medicament according to one of Claims 1 to 14, characterized in that the ibuprofen is present as a pseudoracemate or as a mixture of the racemate with its enantiomers.

17. Medicament according to one of Claims 1 to 14, characterized in that the ibuprofen is present as pure S- or R-ibuprofen.

18. Medicament according to one of Claims 1 to 14, characterized in that the ibuprofen is present as a mixture of different proportions of S- and R-enantiomers.

19. Use of a pharmaceutically administrable nanosol of ibuprofen which is present to at least 50% as R-ibuprofen, which
a) has an inner phase of ibuprofen which has a particle size of 10 - 800 nm and possesses a surface charge,
b) an outer phase of gelatin, a collagen hydrolysate or a gelatin derivative, which is oppositely charged, and
c) an approximate or complete isoionic state of charge of the inner and outer phase and
d) is physiologically absorbable,
for the preparation of medicaments having immediate analgesic and/or antirheumatic, and also antipyretic action.

20. Use of a pharmaceutically administrable nanosol of ibuprofen which is present to more than 50% as S-ibuprofen, which
a) has an inner phase of ibuprofen which has a particle size of 10 - 800 nm and possesses a surface charge,
b) an outer phase of gelatin, a collagen hydrolysate or a gelatin derivative, which is oppositely charged, and
c) an approximate or complete isoionic state of charge of the inner and outer phase and
d) is physiologically absorbable,
for the preparation of medicaments having immediate analgesic and/or antirheumatic, and also antipyretic action.

21. Use of a pharmaceutically administrable nanosol of ibuprofen which is present to at least 50% as S- or R-ibuprofen, which
a) has an inner phase of ibuprofen which has a particle size of 10 - 800 nm and possesses a surface charge,
b) an outer phase of gelatin, a collagen hydrolysate or a gelatin derivative, which is oppositely charged, and
c) an approximate or complete isoionic state of charge of the inner and outer phase and
d) is physiologically absorbable,
for the preparation of medicaments having analgesic and/or antirheumatic immediate and sustained-release action.

## Revendications

1. Médicament à action rapide destiné au traitement de maladies accompagnées de douleur et/ou d'inflammation ainsi que de fièvre, contenant de l'ibuprofène à côté de supports et de substances auxiliaires pharmaceutiques classiques, caractérisé en ce que l'ibuprofène se présente sous forme d'un nanosol pharmaceutiquement applicable, qui contient comme support, principalement de la gélatine, un hydrolysat de collagène ou un dérivé de gélatine, le nanosol présentant
a) une phase interne formée d'ibuprofène, qui présente des diamètres de particules de 10 à 800 nm et qui possède une charge de surface,
b) une phase externe formée de gélatine, d'un hydrolysat de collagène ou d'un dérivé de gélatine, portant une charge opposée
c) un état de charge presque iso-ionique ou tout à fait iso-ionique des phases interne et externe,
et
d) peut être résorbé physiologiquement.

2. Médicament suivant la revendication 1, caractérisé en ce que l'ibuprofène est présent sous forme de nanodispersion aqueuse fluide.

3. Médicament suivant la revendication 1, caractérisé en ce que l'ibuprofène est présent sous forme de nanodispersion solide pouvant être remise en suspension.

4. Médicament suivant l'une des revendications 1 à 3, caractérisé en ce que l'ibuprofène présente un diamètre moyen de particules inférieur à 400 nm.

5. Médicament suivant l'une des revendications 1 à 4, caractérisé en ce que la gélatine présente un maximum de répartition du poids moléculaire en dessous de 10⁵ D et des degrés Bloom de 0 à 240.

6. Médicament suivant l'une des revendications 1 à 5, caractérisé en ce que la gélatine présente un degré Bloom de 0 à 170.

7. Médicament suivant la revendication 6, caractérisé en ce que la gélatine présente une proportion de peptides de 50 à 90 %.

8. Médicament suivant l'une des revendications 1 à 6, caractérisé en ce que la gélatine présente une proportion d'aminoacides dextrogyres inférieure à 20 %.

9. Médicament suivant l'une des revendications 1 à 8, caractérisé en ce que le rapport en poids de la gélatine à la substance active a une valeur de 0,5:1 à 10:1, de préférence de 0,5:1 à 3:1.

10. Médicament suivant l'une des revendications 1 à 9, caractérisé par une phase externe du nanosol qui contient en outre de la polyvinylpyrrolidone dans un rapport en poids de la gélatine à la polyvinylpyrrolidone de 5:1 à 500:1, notamment de 5:1 à 30:1.

11. Médicament suivant l'une des revendications 1 à 10, caractérisé en ce que la forme pharmaceutique est un comprimé se dissolvant rapidement.

12. Médicament suivant l'une des revendications 1 à 10, caractérisé en ce que la forme pharmaceutique consiste en une poudre ou en granulés se dissolvant rapidement.

13. Médicament destiné au traitement de maladies accompagnées de douleur et/ou d'inflammation ainsi que de fièvre, contenant de l'ibuprofène à côté de supports et de substances auxiliaires pharmaceutiques classiques, caractérisé en ce qu'il se présente en partie sous la forme à action rapide et en partie sous la forme à action retardée avec de l'ibuprofène sous forme d'un nanosol pharmaceutiquement applicable, le nanosol présentant
a) une phase interne formée d'ibuprofène, qui présente des diamètres de particules de 10 à 800 nm et qui possède une charge de surface,
b) une phase externe formée de gélatine, d'un hydrolysat de collagène ou d'un dérivé de gélatine, portant une charge opposée
c) un état de charge presque iso-ionique ou tout à fait iso-ionique des phases interne et externe,
et
d) peut être résorbé physiologiquement.

14. Médicament suivant la revendication 13, caractérisé en ce que la forme pharmaceutique est une capsule de gélatine dure qui contient une poudre se dissolvant rapidement avec un comprimé se dissolvant lentement.

15. Médicament suivant l'une des revendications 1 à 14, caractérisé en ce que l'ibuprofène se présente sous forme de racémate.

16. Médicament suivant l'une des revendications 1 à 14, caractérisé en ce que l'ibuprofène se présente sous forme de pseudoracémate ou de mélange du racémate avec ses énantiomères.

17. Médicament suivant l'une des revendications 1 à 14, caractérisé en ce que l'ibuprofène se présente sous forme de S- ou de R-ibuprofène pur.

18. Médicament suivant l'une des revendications 1 à 14, caractérisé en ce que l'ibuprofène se présente sous forme d'un mélange de différentes proportions de S- et de R-énantiomères.

19. Utilisation d'un nanosol d'ibuprofène pharmaceutiquement applicable, qui est au moins à 50 % sous forme de R-ibuprofène, qui présente
a) une phase interne formée d'ibuprofène, qui présente des diamètres de particules de 10 à 800 nm et qui possède une charge de surface,
b) une phase externe formée de gélatine, d'un hydrolysat de collagène ou d'un dérivé de gélatine, portant une charge opposée
c) un état de charge presque iso-ionique ou tout à fait iso-ionique des phases interne et externe,
et
d) peut être résorbé physiologiquement,
pour la préparation de médicaments à action analgésique et/ou anti-rhumatismale ainsi qu'antipyrétique rapide.

20. Utilisation d'un nanosol d'ibuprofène pharmaceutiquement applicable, qui est à plus de 50 % sous forme de S-ibuprofène, qui présente
a) une phase interne formée d'ibuprofène, qui présente des diamètres de particules de 10 à 800 nm et qui possède une charge de surface,
b) une phase externe formée de gélatine, d'un hydrolysat de collagène ou d'un dérivé de gélatine, portant une charge opposée
c) un état de charge presque iso-ionique ou tout à fait iso-ionique des phases interne et externe,
et
d) peut être résorbé physiologiquement,
pour la préparation de médicaments à action analgésique et/ou anti-rhumatismale ainsi qu'antipyrétique rapide.

21. Utilisation d'un nanosol d'ibuprofène pharmaceutiquement applicable, qui est au moins à 50 % de S- ou de R-ibuprofène, qui présente
a) une phase interne formée d'ibuprofène, qui présente des diamètres de particules de 10 à 800 nm et qui possède une charge de surface,
b) une phase externe formée de gélatine, d'un hydrolysat de collagène ou d'un dérivé de gélatine, portant une charge opposée et
c) un état de charge presque iso-ionique ou tout à fait iso-ionique des phases interne et externe,
et
d) peut être résorbé physiologiquement,
pour la préparation de médicaments à action analgésique et/ou anti-rhumatismale rapide et retardée.
